# EUROPEAN PATENT APPLICATION

(11) **EP 2 933 335 A1**
(43) Date of publication of application: **21.10.2015**
(21) Application number: 14165329.5
(22) Date of filing: 18.04.2014
(51) Int. Cl.: C12N 15/86, A61K 48/00

(54) **A method of treating peripheral neuropathies and motor neuron diseases**

(71) Applicant: GENETHON, 91002 Evry Cedex (FR); Wake Forest University Health Sciences, Winston-Salem, NC 27157 (US)
(72) Inventor: Buj Bello, Ana Maria, 75005 Paris (FR); Childers, Martin, K., Edmonds, WA Washington 98026 (US)
(74) Representative: Cabinet Laurent & Charras

(57) **Abstract**

A composition comprising a molecule for use in the delivery of the molecule to the peripheral nervous system (PNS) and/or to the central nervous system (CNS), wherein the composition is administered by regional infusion.

SEQUENCE LISTING

<110> GENETHON
      WAKE FOREST UNIVERSITY HEALTH SCIENCES

<120> A METHOD OF TREATING PERIPHERAL NEUROPATHIES AND MOTOR NEURON DISEASES

<130> G143-B-41343 EP

<160> 6

<170> BiSSAP 1.2


<210> 1
<211> 23
<212> DNA
<213> Artificial Sequence

<220>
<221> source
<222> 1..23
<223> /mol_type="unassigned DNA"
      /note="MTM1 forward"
      /organism="Artificial Sequence"

<400> 1
ataagttttg gacataagtt tgc                                              23

<210> 2
<211> 20
<212> DNA
<213> Artificial Sequence

<220>
<221> source
<222> 1..20
<223> /mol_type="unassigned DNA"
      /note="MTM1 reverse"
      /organism="Artificial Sequence"

<400> 2
catttgccat acacaatcaa                                                  20


<210> 3
<211> 19
<212> DNA
<213> Artificial Sequence

<220>
<221> source
<222> 1..19
<223> /mol_type="unassigned DNA"
      /note="MTM1 probe"
      /organism="Artificial Sequence"

<400> 19
cgacgctgac cggtctcct                                                   19


<210> 4
<211> 20
<212> DNA
<213> Artificial Sequence

<220>
<221> source
<222> 1..20

<223> /mol_type="unassigned DNA"
      /note="GLU forward"
      /organism="Artificial Sequence"

<400> 4
acgctgattg ctcacaccaa                                                   20


<210> 5
<211> 22
<212> DNA
<213> Artificial Sequence

<220>
<221> source
<222> 1..22
<223> /mol_type="unassigned DNA"
      /note="GLU reverse"
      /organism="Artificial Sequence"

<400> 5
ccccaggtct gcttcatagt tg                                                22


<210> 6
<211> 21
<212> DNA
<213> Artificial Sequence

<220>
<221> source
<222> 1..21
<223> /mol_type="unassigned DNA"
      /note="GLU probe"
      /organism="Artificial Sequence"

<400> 6
cccggcccgt gacctttgtg a                                                 21

## Description

### BACKGROUND OF THE INVENTION

The peripheral nervous system (PNS) consists of nerves and neurons, including peripheral nerves and neuronal ganglia that are located outside the central nervous system (CNS) or extended outside the CNS from the brain and spinal cord.

PNS is involved in numerous neurological disorders, inherited or acquired, such as Charcot-Marie-Tooth disease, diabetic, infectious, toxic and drug-related, or immune-related neuropathies.

Effective clinical interventions for those diseases are very limited. Gene therapy represents a novel therapeutic strategy for the PNS diseases. However, efficient gene transfer of the PNS remains critical for gene therapy of inherited and acquired peripheral neuropathies.

It has been reported that adeno-associated virus (AAV) vectors can efficiently transduce dorsal root ganglion (DRG) neurons. However, it needs a delicate microneurosurgical technique to deliver AAV to the DRG (Glatzel et al., 2000, Proc. Natl. Acad. Sci. U.S.A. 97, 442-447).

Foust et al. (2008, Hum. Gen. Ther. 19, 61-70) reported that AAV could transduce nerve fibers in the dorsal horn and column, indicating DRG transduction, when AAV serotype 8 vector was systemically delivered into neonatal mice. However, the systemic route is not specific and requires large amounts of therapeutic gene.

Zheng et al. (2010, Hum. Gen. Ther. 21, 87-97) reported the capacity of AAV8 in transducing PNS in neonatal mice by intraperitoneal injection and in adult mice by intramuscular injection, in tibialis anterior and gastrocnemius muscles of the hind leg. In both cases, efficient and long-term gene transfer was found in the white matter of the spinal cord, DRG neurons and peripheral nerves. These results support the mechanism of retrograde transport of AAV vectors from muscle to the spinal cord, rather than blood-brain barrier crossing. However, intramuscular delivery requires multiple injection sites associated with a lower efficiency.

Homs et al. (2011, Gene Therapy 18, 622-630) reported the tropism and transduction efficiency of different AAV pseudotypes after sciatic nerve injection. It was observed that AAV8 allows the specific transduction of Schwann cells, offering a gene therapy strategy for peripheral nerve regeneration. However, the local administration in a nerve is not applicable at the clinical level, especially for safety reasons.

Therefore, there is a need in the art for effective, simple and minimally invasive delivery methods to the PNS. The present invention satisfies this unmet need.

### DESCRIPTION OF THE INVENTION

The present invention is based on the observation that, one year after loco-regional infusion of an AAV8 vector in the saphenous vein of a dog, a high amount of said vector was detected in the infused sciatic nerve.

In an unexpected manner, this route of administration which was foreseen for muscular delivery has been shown to be very efficient for delivery to the PNS and/or the CNS.

### Definitions

The articles "a" and "an" are used herein to refer to one or to more than one (*i.e.,* to at least one) of the grammatical object of the article. By way of example, "an element" means one element or more than one element.

"About" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

The term "abnormal" when used in the context of organisms, tissues, cells or components thereof, refers to those organisms, tissues, cells or components thereof that differ in at least one observable or detectable characteristic (e.g., age, treatment, time of day, etc.) from those organisms, tissues, cells or components thereof that display the "normal" (expected) respective characteristic. Characteristics which are normal or expected for one cell or tissue type, might be abnormal for a different cell or tissue type.

A "disease" is a state of health of an animal wherein the animal cannot maintain homeostasis, and wherein if the disease is not ameliorated then the animal's health continues to deteriorate. In contrast, a "disorder" in an animal is a state of health in which the animal is able to maintain homeostasis, but in which the animal's state of health is less favorable than it would be in the absence of the disorder. Left untreated, a disorder does not necessarily cause a further decrease in the animal's state of health.

A disease or disorder is "alleviated" if the severity of a symptom of the disease or disorder, the frequency with which such a symptom is experienced by a patient, or both, is reduced. A disease or disorder is "cured" if the severity of a symptom of the disease or disorder, the frequency with which such a symptom is experienced by a patient, or both, is eliminated.

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

An "effective amount" or "therapeutically effective amount" of a compound is that amount of compound which is sufficient to provide a beneficial effect to the subject to which the compound is administered. An "effective amount" of a delivery vehicle is that amount sufficient to effectively bind or deliver a compound.

"Expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an in vitro expression system. Expression vectors include all those known in the art, such as cosmids, plasmids (e.g., naked or contained in liposomes) and viruses *(e.g.,* lentiviruses, retroviruses, adenoviruses, and adeno-associated viruses) that incorporate the recombinant polynucleotide.

"Homologous" refers to the sequence similarity or sequence identity between two polypeptides or between two nucleic acid molecules. When a position in both of the two compared sequences is occupied by the same base or amino acid monomer subunit, e.g., if a position in each of two DNA molecules is occupied by adenine, then the molecules are homologous at that position. The percent of homology between two sequences is a function of the number of matching or homologous positions shared by the two sequences divided by the number of positions compared X 100. For example, if 6 of 10 of the positions in two sequences are matched or homologous then the two sequences are 60% homologous. Generally, a comparison is made when two sequences are aligned to give maximum homology.

"Isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

In the context of the present invention, the following abbreviations for the commonly occurring nucleic acid bases are used. "A" refers to adenosine, "C" refers to cytosine, "G" refers to guanosine, "T" refers to thymidine, and "U" refers to uridine.

Unless otherwise specified, a "nucleotide sequence encoding an amino acid sequence" includes all nucleotide sequences that are degenerate versions of each other and that encode the same amino acid sequence. The phrase nucleotide sequence that encodes a protein or an RNA may also include introns to the extent that the nucleotide sequence encoding the protein may in some version contain an intron(s).

The terms "patient," "subject," "individual," and the like are used interchangeably herein, and refer to any animal, or cells thereof whether *in vitro* or *in situ,* amenable to the methods described herein. In certain non-limiting embodiments, the patient, subject or individual is a human.

The term "polynucleotide" as used herein is defined as a chain of nucleotides. Furthermore, nucleic acids are polymers of nucleotides. Thus, nucleic acids and polynucleotides as used herein are interchangeable. One skilled in the art has the general knowledge that nucleic acids are polynucleotides, which can be hydrolyzed into the monomeric "nucleotides." The monomeric nucleotides can be hydrolyzed into nucleosides. As used herein polynucleotides include, but are not limited to, all nucleic acid sequences which are obtained by any means available in the art, including, without limitation, recombinant means, i.e., the cloning of nucleic acid sequences from a recombinant library or a cell genome, using ordinary cloning technology and PCR and the like, and by synthetic means.

As used herein, the terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. The polypeptides include natural peptides, recombinant peptides, synthetic peptides, or a combination thereof.

The term "promoter" as used herein is defined as a DNA sequence recognized by the synthetic machinery of the cell, or introduced synthetic machinery, required to initiate the specific transcription of a polynucleotide sequence.

As used herein, the term "promoter/regulatory sequence" means a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

A "constitutive" promoter is a nucleotide sequence which, when operably linked with a polynucleotide which encodes or specifies a gene product, causes the gene product to be produced in a cell under most or all physiological conditions of the cell.

An "inducible" promoter is a nucleotide sequence which, when operably linked with a polynucleotide which encodes or specifies a gene product, causes the gene product to be produced in a cell substantially only when an inducer which corresponds to the promoter is present in the cell.

A "tissue-specific" promoter is a nucleotide sequence which, when operably linked with a polynucleotide encodes or specified by a gene, causes the gene product to be produced in a cell substantially only if the cell is a cell of the tissue type corresponding to the promoter.

A "therapeutic" treatment is a treatment administered to a subject who exhibits signs of pathology, for the purpose of diminishing or eliminating those signs.

As used herein, "treating a disease or disorder" means reducing the frequency with which a symptom of the disease or disorder is experienced by a patient. Disease and disorder are used interchangeably herein.

The phrase "therapeutically effective amount," as used herein, refers to an amount that is sufficient or effective to prevent or treat (delay or prevent the onset of, prevent the progression of, inhibit, decrease or reverse) a disease or condition, including alleviating symptoms of such diseases.

To "treat" a disease as the term is used herein, means to reduce the frequency or severity of at least one sign or symptom of a disease or disorder experienced by a subject.

A "vector" is a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid or a virus. The term should also be construed to include non-plasmid and non-viral compounds which facilitate transfer of nucleic acid into cells, such as, for example, polylysine compounds, liposomes, and the like. Examples of viral vectors include, but are not limited to, adenoviral vectors, adeno-associated virus vectors, retroviral vectors, and the like.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

### Description

The present invention relates to a strategy of delivering a molecule to the peripheral nervous system (PNS) and/or to the central nervous system (CNS) of a subject. By using a marker, e.g. a GFP protein, the methods disclosed herein provide the potential to image and therefore visualize tissues. Alternatively, by delivering a therapeutic molecule to a tissue, a disease or a disorder of the PNS and/or the CNS may be treated.

Available modes of administration of the compounds disclosed herein include enteral administration, parenteral administration, oral administration, intravenous administration, intra-arterial administration, and inhalational administration.

According to the invention, the composition comprising the desired molecule is administered to an isolated limb (loco-regional) by infusion or perfusion. In other words, the invention comprises the regional delivery of the composition in a leg and/or arm by an intravascular route of administration, i.e. a vein (transveneous) or an artery, under pressure. This is usually achieved by using a tourniquet to temporarily arrest blood circulation while allowing a regional diffusion of the infused product, as e.g. disclosed by Petrov et al. (2011, Methods Mol Biol 709:277-86), Arruda et al. (2010, Blood 115(23):4678-88) and Zheng et al. (2012, Molecular Therapy 20(2), 456-461).

In one embodiment, the composition is injected in a limb of the subject. In one embodiment, the subject is a mammal, preferably a dog or a human. When the subject is a human, the limb can be the arm or the leg. When the subject is an animal, the limb can be the upper limb or the lower limb. According to one embodiment, the composition is administered in the lower part of the body of the subject, e.g. in the groin or below the knee.

In one embodiment, the composition is administered to a peripheral vein, advantageously the sapheneous vein, more advantageously the distal saphenous vein. More generally, the composition can be injected in:
- the leg via: the superficial veins (ie. great and small saphenous); the deep veins (ie. femoral, popliteal, anterior or posterior tibial veins); the arteries (ie. femoral, deep femoral, popliteal, anterior or posterior tibial arteries);
- the arm via: the superficial veins (ie digital, metacarpal, cephalic, basilic, median antibrachial veins); the deep veins (ie. digital, metacarpal, radial, ulnar, brachial veins); the arteries (brachial, radial and ulnar arteries and branches).

The volume of the composition to be infused can be up to 50% of the limb volume but can be in a range that varies between about 5 and 20% of the limb volume.

The typical dose in dogs or humans can vary between 10 and 20 ml/kg of body weight, and the dose is typically calculated to be 15 ml/kg of body weight.

The composition comprising the molecule of interest is preferably a saline composition, advantageously a phosphate buffered saline (PBS) composition, e.g. 0.9% saline.

In one embodiment, the pressure to be applied (tourniquet pressure or maximum line pressure) is below 100 000 Pa, advantageously below 50 000 Pa. In a preferred embodiment, the pressure applied is around 300 torr (40 000 Pa).

In one embodiment, the blood circulation of the limb is stopped using a tourniquet that is tightened for several minutes, typically between about 1 and 20 minutes, for example about 15 minutes. In a preferred embodiment, the tourniquet was applied before and during the administration, for example about 10 minutes prior to and about 5 minutes during the infusion. More generally, the pressure is applied for several minutes, typically between about 1 and 20 minutes, for example about 15 minutes. In a preferred embodiment, the pressure is applied before and during the administration, for example about 10 minutes prior to and about 5 minutes during the infusion.

In one embodiment, the average flow rate is comprised between 50 and 150 ml/min, advantageously between 60 and 80 ml/min.

According to a particular embodiment, a tourniquet is positioned at the level of the groin and adjusted until the femoral pulse is no longer detectable by ultrasound to transiently block blood inflow to the target limb. A tight extensible wrap is applied in a distal to proximal direction exsanguinated the limb before the tourniquet is tightened. Vector is suspended in PBS at 20% of the total hind limb volume (determined by water volume displacement) and administered via a 14 gauge catheter placed into a distal branch of the peripheral saphenous vein on the dorsum of the paw. The tourniquet is tightened for a total of 15 minutes (10 minutes prior to and 5 minutes during the infusion).

In one embodiment, the invention provides a method for delivering a molecule to the peripheral nervous system (PNS) and/or to the central nervous system (CNS) of a subject comprising administrating to said subject, by regional or loco-regional infusion, a composition comprising the molecule. In other words, the invention relates to a composition comprising a molecule for use in the delivery of the molecule to the peripheral nervous system (PNS) and/or to the central nervous system (CNS), wherein the composition is administered by regional or loco-regional infusion.
In a preferred embodiment, the composition comprises an effective amount of the molecule.

In the frame of the invention, the peripheral nervous system (PNS) includes the nerves and neurons that are located outside the central nervous system (CNS) or extended outside the CNS from the brain and spinal cord. It includes peripheral nerves (ie. sciatic nerve...), neuronal ganglia (ie. dorsal root ganglia...) and neurons in the spinal cord (motoneurons).

In the frame of the invention, the central nervous system (CNS) includes the spinal cord, in particular motor neurons (or motoneurons).

In an unexpected manner, it was observed that the molecule delivered by the method of the invention could be detected in the target tissues 1 year after a single loco-regional infusion. In one embodiment, the present invention provides a method for delivering a molecule to the peripheral nervous system (PNS) and/or to the central nervous system (CNS) of a subject, wherein the molecule is detected in the PNS and/or the CNS of the subject for 1 day, 3 days, 1 week, 2 weeks, 1 month, 3 months, 6 months, 1 year, 5 years or longer. However, even a transient expression/detection may be useful for imaging of tissues, or for treatment of a subject in need thereof.

In a specific embodiment, the method comprises a single administration of the composition.

The invention contemplates delivery of any type or class of molecule using the methods disclosed herein. The molecule may be a chemical molecule, an antibody, a peptide or a protein, a nucleic acid, or a vector, for example a viral vector. The molecule also may be one designed for labeling and imaging, or it may be a therapeutic molecule.

In one embodiment, for labeling or diagnostic purposes, the molecule is any molecule which allows the visualization, advantageously the specific and selective visualization of the target tissues, using the available detection and imaging techniques (radioactivity, fluorescence, MRI, ...). Non-limiting examples of such molecules include, a contrast agent, a fluorophore, or a fluorescently labeled imaging agent.
Of special interest is a therapeutic molecule, able to cure or alleviate a disease or a disorder of the PNS and/or of the CNS. In one embodiment, said disease is associated with one or more defective proteins.

In this context, the therapeutic molecule can be:
- A biologically functional protein or an active fragment thereof. As would be understood in the art, an active fragment is a portion or portions of a full length sequence that retain the biological function of the full length sequence;
- An isolated nucleic acid sequence encoding said protein or fragment, i.e. a transgene, nude or harbored by an expression vector. More generally, it can be an isolated nucleic acid encoding a peptide having substantial homology to the peptides disclosed herein. Preferably, the nucleotide sequence of an isolated nucleic acid encoding a peptide of the invention is "substantially homologous", that is, is about 60% homologous, more preferably about 70% homologous, even more preferably about 80% homologous, more preferably about 90% homologous, even more preferably, about 95% homologous, and even more preferably about 99% homologous to a nucleotide sequence of an isolated nucleic acid encoding said protein or fragment;
- An isolated nucleic acid sequence that is capable of correcting a defect in a native protein, e.g. an antisense RNA (siRNA, shRNA) inducing exon skipping/inclusion or silencing gene expression, a microRNA (miRNA), other RNA and DNA fragments.

In one embodiment, the isolated nucleic acid sequence encodes a protein where the nucleic acid is referred to as a transgene. In a particular embodiment, said transgene corresponds to an open reading frame and is delivered by the method of the invention, possibly via an expression vector.

In one embodiment, the sequence of the transgene corresponds to a native (endogenous) sequence present in the subject. In a specific embodiment, the endogenous sequence is defective, i.e. displays one or more mutations leading to the lack of the corresponding protein or the production of a partially or fully inactive protein, or to the corresponding protein with a gain-of-function, notably in the PNS and/or CNS, and is associated with a disease.

More generally, the molecule of interest can be a therapeutic protein or a sequence encoding said protein as disclosed above.
Other molecules of interest include neurotrophic factors (NGF, BDNF, NT3, CNTF, GDNF, neurturin, persephin, artemin, ...), trophic factors (IGF1, IGF2, ...), apoptosis or cell death-inducing genes (e.g. caspases).

The nucleic acid sequence can be single- or double-stranded DNA, RNA or cDNA.

In one embodiment, the nucleic acid is administered as a naked nucleic sequence. In order to facilitate the cell transduction, the nucleic acid sequence can be associated with various structures such as, for example, systems for colloidal dispersions (nanocapsules, microspheres, ...) or lipid-based systems (emulsions, micelles, liposomes, ...).

In another embodiment, the composition comprises a plasmid or a vector. According to a specific embodiment, the isolated nucleic acid is inserted into the vector. In brief summary, the expression of natural or synthetic nucleic acids is typically achieved by operably linking a nucleic acid or portions thereof to a promoter, and incorporating the construct into an expression vector. The vectors to be used are suitable for replication and, optionally, integration in eukaryotic cells. Typical vectors contain transcription and translation terminators, initiation sequences, and promoters useful for regulation of the expression of the desired nucleic acid sequence.

In one embodiment, the composition comprises an expression vector, advantageously a viral vector. In one embodiment, the viral vector is selected from the group consisting of a baculoviral vector, herpes viral vector, lentiviral vector, retroviral vector, adenoviral vector, and adeno-associated viral (AAV) vector, advantageously an AAV vector.

Adeno-associated viral (AAV) vectors have become powerful gene delivery tools for the treatment of various disorders. AAV vectors possess a number of features that render them ideally suited for gene therapy, including a lack of pathogenicity, minimal immunogenicity, and the ability to transduce postmitotic cells in a stable and efficient manner. Expression of a particular gene contained within an AAV vector can be specifically targeted to one or more types of cells by choosing the appropriate combination of AAV serotype, promoter, and delivery method.

In one embodiment, the encoding sequence is contained within an AAV vector. More than 100 naturally occurring serotypes of AAV are known. Many natural variants in the AAV capsid exist, allowing identification and use of an AAV with properties specifically suited for PNS and/or CNS. AAV viruses may be engineered using conventional molecular biology techniques, making it possible to optimize these particles for cell specific delivery of nucleic acid sequences, for minimizing immunogenicity, for tuning stability and particle lifetime, for efficient degradation, for accurate delivery to the nucleus.

As mentioned above, the use of AAVs is a common mode of exogenous delivery of DNA as it is relatively non-toxic, provides efficient gene transfer, and can be easily optimized for specific purposes. Among the serotypes of AAVs isolated from human or non-human primates (NHP) and well characterized, human serotype 2 is the first AAV that was developed as a gene transfer vector. Other currently used AAV serotypes include AAV1, AAV3, AAV4, AAV5, AAV6, AAV7, AAV8, AAV9, AAV10, AAV11 and AAV12. In addition, non-natural engineered variants and chimeric AAV can also be useful.

Desirable AAV fragments for assembly into vectors include the cap proteins, including the vp1, vp2, vp3 and hypervariable regions, the rep proteins, including rep 78, rep 68, rep 52, and rep 40, and the sequences encoding these proteins. These fragments may be readily utilized in a variety of vector systems and host cells.

Such fragments may be used alone, in combination with other AAV serotype sequences or fragments, or in combination with elements from other AAV or non-AAV viral sequences. As used herein, artificial AAV serotypes include, without limitation, AAV with a non-naturally occurring capsid protein. Such an artificial capsid may be generated by any suitable technique, using a selected AAV sequence (e.g., a fragment of a vp1 capsid protein) in combination with heterologous sequences which may be obtained from a different selected AAV serotype, non-contiguous portions of the same AAV serotype, from a non-AAV viral source, or from a non-viral source. An artificial AAV serotype may be, without limitation, a chimeric AAV capsid, a recombinant AAV capsid, or a "humanized" AAV capsid. Thus exemplary AAVs, or artificial AAVs, include AAV2/8 (US 7,282,199), AAV2/5 (available from the National Institutes of Health), AAV2/9 (WO2005/033321), AAV2/6 (US 6,156,303), and AAVrh8 (WO2003/042397), among others. In one embodiment, the vectors useful in the compositions and methods described herein contain, at a minimum, sequences encoding a selected AAV serotype capsid, e.g., an AAV8 capsid, or a fragment thereof. In another embodiment, useful vectors contain, at a minimum, sequences encoding a selected AAV serotype rep protein, e.g., AAV8 rep protein, or a fragment thereof. Optionally, such vectors may contain both AAV cap and rep proteins. In vectors in which both AAV rep and cap are provided, the AAV rep and AAV cap sequences can both be of one serotype origin, e.g., all AAV8 origin. Alternatively, vectors may be used in which the rep sequences are from an AAV serotype which differs from that which is providing the cap sequences. In one embodiment, the rep and cap sequences are expressed from separate sources (e.g., separate vectors, or a host cell and a vector). In another embodiment, these rep sequences are fused in frame to cap sequences of a different AAV serotype to form a chimeric AAV vector, such as AAV2/8 (US 7,282,199).

Advantageously, the nucleic acid sequence of interest is inserted between the ITR (« Inverted Terminal Repeat ») sequences of the AAV vector.

As known in the art, recombinant viral particles can be obtained, e.g. by tri-transfection of 293 HEK cells, by the herpes simplex virus system and by the baculovirus system. The vector titers are usually expressed as viral genomes per ml (vg/ml).

In one embodiment, the expression vector comprises regulatory sequences, especially a promoter sequence. Such promoters can be natural or synthetic (artificial) promoters, inducible or constitutive.

In one embodiment, the promoter is an ubiquitous promoter or having a low tissue-specificity. As an example, the expression vector can harbor the phosphoglycerate kinase 1 (PGK), EF1, β-actin, CMV promoter.

In a preferred embodiment, the promoter sequence is chosen in order to adequately govern the expression of the nucleic acid sequence placed under its control, in terms of expression level but also of tissue specificity. In one embodiment, the expression vector comprises a PNS and/or CNS specific promoter, such as the P0, NSE, SYN1, Hb9 or Thy-1 promoter.

A non-exhaustive list of other possible regulatory sequences is:
- a polyadenylation signal, e.g. the polyA of the gene of interest, the polyA of SV40 or of beta hemoglobin (HBB2), advantageously in 3' of the sequence of interest ;
- sequences for transcript stabilization, e.g. intron 1 of hemoglobin (HBB2);
- enhancer sequences ;
- miRNAs target sequences.

To date, more than 40 genes have been involved in Charcot-Marie-Tooth neuropathies, and more than 15 genes in diseases affecting motoneurons. Among the proteins of interest which defect is involved in a disease of the PNS and/or CNS are:
- Charcot-Marie-Tooth neuropathies: *PMP22, GJB1, MPZ, LITAF, EGR2, NEFL, GAN1, KIF1B, MFN2, TRPV4, GDAP1, DYNC1H1, RSAM1, GNB4, HSPB1, HSPB3, HSPB8, GARS, YARS, AARS, HARS, KARS, MTMR2, MTMR13, RAB7, SPTLC1, SPTLC2, DNM2, PDK3, SH3TC2, NDRG1, PRX, HK1, FGD4, FIG4, CTDP1, LMNA, MED25, PRPS1, FBLN5, INF2, BSCL2, DCTN1, SLC5A7, SETX, REEP1, IGHMPB2, ATP7A;*
- Motoneuron diseases: *SMN1, SOD1, TARDBP, FUS, C9ORF72, SETX, VAPB. ANG, FIG 4, OPTN, VCP, alsin, spatacsin, UBQLN2, SIGMAR1, DCTN1.*

As an example, the myotubularin (MTM1) gene family comprises 15 members, and mutations in two members (MTMR2, MTMR13) are associated with diseases of the PNS. In this context, the delivery of the functional, possibly native protein, or of the corresponding gene should treat these diseases or even cure them.

According to another aspect, the present invention provides a method for treating a disease of the peripheral nervous system (PNS) and/or of the central nervous system (CNS) in a subject comprising administrating to said subject, by regional or loco-regional infusion, a composition comprising a therapeutic molecule. In other words, the present invention concerns a composition comprising a therapeutic molecule for use in treating a disease of the peripheral nervous system (PNS) and/or of the central nervous system (CNS), wherein the composition is administered by loco-regional infusion. In a preferred embodiment, the composition comprises a therapeutically effective amount of the molecule.

Of specific interest are the peripheral neuropathies and the motor neuron diseases. Inherited as well as acquired diseases are concerned. Examples of inherited peripheral neuropathies are Charcot-Marie-Tooth (CMT) neuropathies. Examples of neuromuscular disorders with motoneuron (CNS) involvement are spinal muscular atrophy (SMA) and amyotrophic lateral sclerosis (ALS).

Charcot-Marie-Tooth neuropathies include: demyelinating CMT (AD-CMT1 and CMT4 forms), axonal CMT (AD-CMT2 and AR-CMT2 forms), intermediate CMT (DI-CMT forms), X-linked CMT (D-CMTX and R-CMTX forms), CMT 'plus', dHMN (AD-HMN, R-HMN, X-HMN forms). In this list, A means "autosomal", X means "X-linked", R means "recessive", D means "dominant".

According to the present invention, the composition comprises at least one active molecule, possibly different molecules. Such a composition can also include a pharmaceutically acceptable inert vehicle. Various excipients, stabilizers and other suitable compounds known to those skilled in the art can be envisaged in such a composition.

Since the composition according to the invention is to be administered by loco-regional infusion, it will preferably be in liquid form. Determining the vector concentration, the amount to be injected and the frequency of injections is part of normal practice for those skilled in the art.

According to another aspect, the present invention concerns a kit comprising a composition as defined above and any piece of material dedicated to the loco-regional infusion, advantageously a tourniquet system and/or a device for monitoring the pressure applied at the site of injection or the pulse of the infused vein or artery (e.g. ultrasound device).

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, "Molecular Cloning: A Laboratory Manual", fourth edition (Sambrook, 2012); "Oligonucleotide Synthesis" (Gait, 1984); "Culture of Animal Cells" (Freshney, 2010); "Methods in Enzymology" "Handbook of Experimental Immunology" (Weir, 1997); "Gene Transfer Vectors for Mammalian Cells" (Miller and Calos, 1987); "Short Protocols in Molecular Biology" (Ausubel, 2002); "Polymerase Chain Reaction: Principles, Applications and Troubleshooting", (Babar, 2011); "Current Protocols in Immunology" (Coligan, 2002). These techniques are applicable to the production of the polynucleotides and polypeptides of the invention, and, as such, may be considered in making and practicing the invention. Particularly useful techniques for particular embodiments will be discussed in the sections that follow.

It is to be understood that wherever values and ranges are provided herein, all values and ranges encompassed by these values and ranges, are meant to be encompassed within the scope of the present invention. Moreover, all values that fall within these ranges, as well as the upper or lower limits of a range of values, are also contemplated by the present application.

The following examples further illustrate aspects of the present invention. However, they are in no way a limitation of the teachings or disclosure of the present invention as set forth herein.

### EXPERIMENTAL EXAMPLES

The invention is further described in detail by reference to the following experimental examples. These examples are provided for purposes of illustration only, and are not intended to be limiting unless otherwise specified.

Without further description, it is believed that one of ordinary skill in the art can, using the preceding description and the following illustrative examples, make and utilize the compounds of the present invention and practice the claimed methods. The following working examples therefore, specifically point out the preferred embodiments of the present invention, and are not to be construed as limiting in any way the remainder of the disclosure.

### Materials and Methods:

### Animals

XLMTM dogs were described previously (Beggs et al., 2010, Proc Natl Acad Sci USA 107(33):14697-702). Affected males were identified by polymerase chain reaction-based genotyping, as described.

### Preparation and administration of rAAV8-MTM1 in dogs

The recombinant adeno-associated virus vector containing a canine myotubularin cDNA regulated by the desmin promoter, rAAV2/8-pDesmin-*MTM1* canine (designated rAAV8*-MTM1),* was produced in a baculovirus/Sf9 system. Two baculovirus batches were generated, one expressing *rep* and *cap* AAV genes and the second bearing the canine *MTM1* cDNA (XM850116, NCBI) downstream from the human desmin promoter (pDesmin). The rAAV*-MTM1* vector particles were produced after baculoviral double infection of insect Sf9 cells and purified from total cell culture using AVB affinity chromatography column (GE Healthcare, AVB Sepharose high performance). The concentration in vg/mL was determined from DNase-resistant particles, as described above. Other routine quality control assays for rAAV vectors were performed, including sterility and purity tests (Yuasa et al., 2007, Gene Ther 14(17): 1249-60).

### Intravenous regional limb infusions

In an anesthetized XLMTM dog, the vector rAAV8-*MTM1* (2.5 x 10¹³ vg/kg) diluted in phosphate buffered saline (PBS) was infused into the distal saphenous vein under pressure (300 torr) against a tourniquet as described (Petrov et al., 2011, Methods Mol Biol 709:277-86; Arruda et al., 2010, Blood 115(23):4678-88). Briefly, a tourniquet was positioned at the level of the groin and adjusted until the femoral pulse was no longer detectable by ultrasound to transiently block blood inflow to the target limb. A tight extensible wrap applied in a distal to proximal direction exsanguinated the limb before the tourniquet was tightened. Vector was suspended in PBS at 20% of the total hind limb volume (determined by water volume displacement) and administered via a 14 gauge catheter placed into a distal branch of the peripheral saphenous vein on the dorsum of the paw. The tourniquet was tightened for a total of 15 minutes (10 minutes prior to and 5 minutes during the infusion). One hind limb was infused with vector whereas the contralateral hind limb was not infused.

### Quantification of the viral titers in the dog tissues

The number of vector genomes per diploid genome was quantified from 80 ng of total DNA by Taqman real-time PCR using a 7900 HT thermocycler (Applied Biosystem, France). The canine β-glucuronidase gene was used for standardization. Primers used for vector genome (*MTM1*) amplification were:
*5'-ATAAGTTTTGGACATAAGTTTGC-3'* (forward; SEQ ID NO: 1),
*5'-CATTTGCCATACACAATCAA-3'* (reverse; SEQ ID NO: 2); and
*5'-CGACGCTGACCGGTCTCCT-3'* (probe; SEQ ID NO: 3).
Primers and probe used for β-glucuronidase amplification were:
*5'-ACGCTGATTGCTCACACCAA-3'* (forward; SEQ ID NO: 4),
*5'-CCCCAGGTCTGCTTCATAGTTG-3' (reverse;* SEQ ID NO: 5); and
*5'-CCCGGCCCGTGACCTTTGTGA-3'* (probe; SEQ ID NO: 6) (Applied Biosystem).

### Results:

### Locoregional infusion of an AAV8 vector leads to increased transduction in peripheral nerves

A tourniquet was placed on the upper part of the left hind limb of a 9 week-old XLMTM dog and a rAAV8-Des-cMTM1 vector (2.5x10¹³vg/kg) was injected under pressure via the saphenous vein.

One year after vector administration, the dog was euthanized and a large panel of tissues and organs were collected for vector biodistribution analysis. Quantification of vector genome copies (vg) per diploid genome (dg) in samples revealed that the infused sciatic nerve was the best transduced tissue of the body, with about ∼15 times more vector DNA (5.2 vg/dg) than in the contralateral nerve (0.3 vg/dg).

### Other Embodiments

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

The disclosures of each and every patent, patent application, and publication cited herein are hereby incorporated herein by reference in their entirety. While this invention has been disclosed with reference to specific embodiments, it is apparent that other embodiments and variations of this invention may be devised by others skilled in the art without departing from the true spirit and scope of the invention. The appended claims are intended to be construed to include all such embodiments and equivalent variations.

## Claims

1. A composition comprising a molecule for use in the delivery of the molecule to the peripheral nervous system (PNS) and/or to the central nervous system (CNS), wherein the composition is administered by regional infusion.

2. A composition comprising a therapeutic molecule for use in treating a disease of the peripheral nervous system (PNS) and/or of the central nervous system (CNS), wherein the composition is administered by regional infusion.

3. A composition for its use according to claim 2, wherein the disease is a peripheral neuropathy or a motor neuron disease.

4. A composition for its use according to any of claims 1 to 3, wherein the molecule is selected from the group consisting of: a chemical molecule, a protein, an antibody, a nucleic acid sequence.

5. A composition for its use according to any of claims 1 to 4, wherein the composition comprises a therapeutic protein or a nucleic acid sequence, advantageously a nucleic acid sequence encoding a therapeutic protein involved in diseases of the PNS and/or the CNS, or an active fragment thereof.

6. A composition for its use according to any of claims 1 to 5, wherein the composition comprises an expression vector, advantageously a viral vector.

7. A composition for its use according to claim 6, wherein the viral vector is selected from the group consisting of a lentiviral vector, retroviral vector, adenoviral vector, and adeno-associated viral (AAV) vector, advantageously an AAV vector.

8. A composition for its use according to claim 7, wherein the viral vector is natural AAV vector having a serotype selected from the group consisting of AAV1, AAV2, AAV3, AAV4, AAV5, AAV6, AAV7, AAV8, AAV9, AAV10, AAV11, AAV12, or engineered artificial AAV serotypes" advantageously AAV8.

9. A composition for its use according to any of claims 1 to 8, wherein the subject is a mammal, advantageously a dog or a human.

10. A composition for its use according to any of claims 1 to 9, wherein the regional infusion is performed in a limb of the subject, advantageously in the lower part of the body of the subject.

11. A composition for its use according to claim 10, wherein the regional infusion is performed via injection in a peripheral vein.

12. A composition for its use according to any of claims 1 to 11, comprising a single administration of the composition.

13. A kit comprising a composition as defined in any of claims 1 to 12 and at least one piece of material dedicated to the regional infusion, advantageously a tourniquet system and/or a device for monitoring the pressure.
